# EUROPEAN PATENT APPLICATION

(11) **EP 2 902 781 A1**
(43) Date of publication of application: **05.08.2015**
(21) Application number: 14000384.9
(22) Date of filing: 03.02.2014
(51) Int. Cl.: G01N 33/50

(54) **Methods for the identification of genes modulating the expression, localization and/or trafficking of integrins**

(71) Applicant: Universität Heidelberg, 69117 Heidelberg (DE)
(72) Inventor: Starkuviene-Erfle, Vytaute, 69239 Neckarsteinach (DE); Erfle, Holger, 69239 Neckarsteinach (DE); Eskova, Anastasia, 72074 Tübingen (DE)
(74) Representative: Müller-Boré & Partner Patentanwälte PartG mbB

(57) **Abstract**

The present invention relates to methods for the identification of protein coding and protein non-coding genes, their products, non-coding RNA species (ncRNAs) and/or chemical agents which modulate the expression, localization and/or trafficking of integrins in any kind of cell. In preferred embodiments, the methods of the present invention are fully automated and are suitable for high-throughput and high-content analysis.

## Description

The present invention relates to methods for the identification of protein coding and protein non-coding genes, their products, non-coding RNA species (ncRNAs) and/or chemical agents which modulate the expression, localization and/or trafficking of integrins in any kind of cell. In preferred embodiments, the methods of the present invention are fully automated and are suitable for high-throughput and high-content analysis.

Integrins are one of the major cell surface adhesion receptors, involved in numerous functions, such as cell migration, signal transduction, proliferation, survival and differentiation. Integrins are heterodimeric complexes composed of α and chains. Currently, 18 integrin α chains and 8 β chains are known in human cells, forming 24 different complexes specific for diverse ligands. Availability and functionality of integrins on the cell surface strongly depends on their biosynthetic trafficking, internalization, re-cycling and degradation.

Newly synthesized integrins are assembled into heterodimers in the endoplasmatic reticulum (ER), traverse the Golgi complex in the inactive state and require binding to the TGN38/46 at the trans-Golgi to efficiently reach the plasma membrane (PM). Both ligand-bound (clustered) and non-bound (non-clustered) surface integrins can be internalized. Clathrin-dependent and clathrin-independent pathways have been described to promote internalization of different integrins. Different from many other endocytic cargoes, integrin internalization via the clathrin-dependent pathway frequently requires CLASPs (for instance, Dab2, Numb or ARH). Most of the internalized integrins are recycled to the PM either through Rab11 (long-loop)- or Rab4 (short-loop)-specific pathways. Ligand-bound integrins can also be directed to lysosomal or non-lysosomal degradation pathways. Competitive binding of diverse regulators to integrins might be needed to promote re-cycling or intracellular retention. The exact pathway of integrin trafficking depends not only on the integrin activation status, but also on cell type, composition of integrin heterodimer and extracellular stimuli.

Trafficking regulators involved in integrin internalization, re-cycling or degradation are starting to emerge. Small GTPases of the Rab family are the best investigated in this context with Rab4, Rab5, Rab7, Rab11, Rab21, and Rab25 known to mark routes of endocytic integrin trafficking. Diverse SNAREs, regulators of membrane fusion, were shown to be important for integrin re-cycling (e.g., SNAP23-syntaxin 4-VAMP3 complex, syntaxin 3, syntaxin 6). Several molecular motors were shown to play a role in integrin trafficking. For instance, actin-based motors, such as myosin 10, regulate integrin-mediated cell adhesion in filopodia, and myosin 6 promotes internalization of ligand-bound integrin α5β1. Microtubule-based motor KIF1C was shown to deliver integrin α5β1 from the perinuclear recycling compartment to the trailing edge of migrating cells. However, a comprehensive view on the molecular machinery regulating integrin trafficking is still missing, especially in different cell lines. The same holds true for the regulation of integrin expression levels which are influenced by numerous intrinsic and extrinsic factors.

As has been stated above, a comprehensive view on the molecular machinery regulating integrin trafficking and expression is still missing. Accordingly, the technical problem underlying the present invention is to provide means for identifying integrin regulators of any nature, *i.e*., proteins, nucleic acids, and/or chemical agents which modulate the expression, localization and/or trafficking of integrins. This should be preferably possible in a rapid, confident and automated manner allowing for high-throughput and high-content analysis.

The solution to the above technical problem is achieved by the embodiments characterized in the claims.

In particular, the present invention relates to a method for the identification of genes, gene products, ncRNAs, and/or chemical agents which modulate the expression, localization and/or trafficking of an integrin of interest, said method comprising the steps:
(a) providing a solid support carrying cells on said solid support in defined locations,
(b1) modulating the expression of one or more gene(s) of interest in said cells, wherein per defined location on the solid support one gene of interest is modulated, and/or
(b2) modulating the activity of one or more gene product(s) of interest in said cells, wherein per defined location on the solid support, the activity of one gene product of interest is modulated, and/or
(b3) modulating the expression of one or more ncRNA(s) of interest in said cells, wherein per defined location on the solid support one ncRNA of interest is modulated, and/or
(b4) applying one or more chemical agent(s) of interest to said cells, wherein per defined location on the solid support one chemical agent of interest is applied,
   wherein any number of the steps (b1) to (b4) can be performed in any order and in any combination,
(c1) determining the amount of intracellular integrin of interest by
   (i) contacting said cells with a primary binding agent which specifically recognizes the integrin of interest,
   (ii) subjecting the cells to conditions that promote the internalization of the integrin of interest,
   (iii) stripping remaining cell surface-bound primary binding agents from the cells,
   (iv) labeling said cells intracellularly with a secondary binding agent which specifically binds to the primary binding agent and carries a fluorescent label, and
   (v) determining the amount of the fluorescent label in the intracellular compartment of the cells in each defined location, wherein the amount of fluorescent label correlates to the amount of intracellular integrin of interest,
   and/or
(c2) determining the amount of surface integrin of interest by
   (i) subjecting the cells to conditions that promote the internalization of the integrin of interest,
   (ii) labeling the surface of said cells with
      (α) a primary binding agent which specifically recognizes the integrin of interest and carries a fluorescent label, or
      (β) a primary binding agent which specifically recognizes the integrin of interest and subsequently with a secondary binding agent which specifically binds to the primary binding agent and carries a fluorescent label,
   (iii) determining the amount of the fluorescent label on the surface of the cells in each defined location, wherein the amount of fluorescent label correlates to the amount of surface integrin of interest,
   and/or
(c3) determining the total amount of integrin of interest by
   (i) contacting said cells with a primary binding agent which specifically recognizes the integrin of interest,
   (ii) subjecting the cells to conditions that promote the internalization of the integrin of interest,
   (iii) labeling said cells intracellularly and on the surface with a secondary binding agent which specifically binds to the primary binding agent and carries a fluorescent label, and
   (iv) determining the total amount of the fluorescent label on the surface of the cells and in the intracellular compartment of the cells in each defined location, wherein the total amount of fluorescent label correlates to the total amount of integrin of interest,
   wherein any number of the steps (c1) to (c3) can be performed in any order and in any combination,
(d) comparing the amount(s) of the fluorescent label determined in steps (c1) to (c3) and/or comparing ratios of said amounts to each other for each defined location with the respective amount(s) of the fluorescent label and/or respective ratios of said amounts determined in control cells in which no modulation of gene expression, no modulation of the activity of a gene product, no modulation of the expression of an ncRNA, and no application of a chemical agent has been performed, and
(e) identifying genes and/or gene products and/or ncRNAs and/or chemical agents which modulate the expression, localization and/or trafficking of the integrin of interest, wherein such genes and/or gene products and/or ncRNAs and/or chemical agents are indicated by a statistically significant difference between the amount(s) of the fluorescent label(s) determined in steps (e1) to (e3) for each defined location and/or ratios of said amounts to each other, and the amount(s) of the fluorescent label(s) and/or respective ratios of said amounts to each other determined in control cells in which no modulation of gene expression, no modulation of the activity of a gene product, no modulation of the expression of an ncRNA, and no application of a chemical agent has been performed in each defined location.

In the context of the present invention, the term "integrin" is not particularly limited, as the method of the present invention is applicable to all 28 mammalian integrins known in the art. In a preferred embodiment, the integrin is the integrin α2β1, preferably human integrin α2β1.

Integrin regulators of any nature, *i.e.,* proteins, nucleic acids, and/or chemical agents which modulate the expression, localization and/or trafficking of integrins, to be identified in the present invention are not particularly limited and include any regulator that might be of interest in the context of integrin expression, localization and/or trafficking. They include genes encoding proteins, as well as genes encoding non-coding RNA (ncRNA).

Accordingly, protein coding and protein non-coding gene products to be identified in the present invention are not particularly limited and include any protein coding and protein non-coding gene products that might be of interest in the context of integrin expression, localization and/or trafficking. They include proteins, peptides, as well as any species of nucleic acids known in the art.

Chemical agents to be identified in the present invention are not particularly limited and include any chemical agents that might be of interest in the context of integrin expression, localization and/or trafficking.

The solid support provided in step (a) of the method of the present invention is not particularly limited, provided it is a solid support that allows for the cultivation, and optionally the attachment, of cells in defined locations of said solid support. Respective solid supports include multiwell plates, microtiter plates, slides, coverslips, micropatterned and/or bioengineered surfaces, and cell arrays, as known in the art. Said solid supports can be based on chemically and/or enzymatically modified or unmodified glass, plastic, or membranes. Further, said solid supports may be uncoated or coated with any component or mixture of components that supports cell attachment, growth, proliferation, transfection, infection or any other desired cellular or organismal function.

Preferred components in this respect are selected from the group consisting of the native or modified (e.g. denatured) components of the extracellular matrix (e.g. fibronectin, collagen I, or gelatin).

The cells to be used in the method of the present invention are not particularly limited, provided they express the integrin of interest. They are intended to include any type of cell. In a particular example, HeLa cells can be used.

Concerning steps (b1), (b2), (b3), and (b4) of the method of the present invention, any one of said steps, any two of said steps, any three of said steps, or all four of said steps can be performed. In case more than one of said steps of performed, said steps can be performed simultaneously (combinatorial assay) or in any particular order.

Modulation of the expression of one or more protein coding or protein non-coding gene(s) of interest or their respective products in said cells can be performed by any method known in the art on the level of DNA, RNA, and protein. Modulation can lead to increased (e.g., over-expression of cDNA) or decreased expression (e.g., by RNA interference) of protein coding or protein non-coding gene(s), or their respective products, to any degree. Modulation can be achieved by any transient or stable modulation method known in the art (e.g., RNA interference, genome or cell engineering). Modulation can be achieved by delivery of one or more protein coding or protein non-coding gene(s), their respective products, ncRNAs or any type of nucleic acids, and/or chemical reagents by any method known in the art (e.g., transfection, microinjection, electroporation).

Modulation of the activity and expression of protein coding and protein-non coding genes of interest in said cells can be performed by any method known in the art. Modulation can change or abolish completely expression level of protein coding and protein-non coding genes directly in their genomic loci, prior and after their transcription by any method known in the art.

Modulation of the activity and expression of any ncRNA species of interest in said cells can be performed by any method known in the art. Modulation can change or abolish completely expression level of ncRNAs directly in their genomic loci, prior and after their transcription by any method known in the art.

Modulation of the activity of one or more protein coding and non-coding gene product(s) of interest in said cells can be performed by any method known in the art. Modulation can change or abolish completely expression level of protein coding and non-coding genes products directly in their genomic loci, prior and after their transcription and translation by any method known in the art. Delivery of one or more antibodies, antibody fragments (e.g., Fab fragments, F(ab')₂ fragments, and Fab' fragments), or antibody mimetics (e.g., single-chain variable fragments (scFv); single-domain antibodies, affibodies, anticalins, DARPins, monobodies, and peptide aptamers) directed against the one or more genes or gene product(s) of interest can be performed by any method known in the art.

Application of one or more chemical agent(s) of interest to the cells can be performed in step (b4) of the method of the present invention by adding said agent(s) to the cells by any method known in the art, or by immobilizing said agent(s) to the solid support prior to the addition of cells to said solid support by any method known in the art. In case of the latter, step (b4) can actually be performed prior or simultaneously to step (a).

According to the present invention, any number of the steps (c1) to (c3) can be performed in any order and in any combination. However, it is preferred that said steps are performed in the order (c1), then (c2), then (c3), in case all three steps are performed. Alternatively, it is preferred that (c2) is performed prior to (c3), in case both steps are performed. In another embodiment, it is preferred that steps (c2) and (c3) are performed prior to step (c1), in case all three steps are performed.

The primary binding agent as used in steps (c1), (c2), and (c3) of the method of the present invention is not particularly limited, provided it is capable of binding to the integrin of interest in a specific manner. Preferably, said binding agent is selected from the group consisting of antibodies, antibody fragments, and antibody mimetics, as defined above, and ligands interacting with the integrin of interest.

In steps (c1), (c2), and (c3) of the method of the present invention, the cells are subjected to conditions that promote the internalization of the integrin of interest, in order to effect the internalization of the surface-bound primary binding agent applied in said steps, or of surface integrin. Suitable conditions for a given type of cell are known in the art. Preferably, cells are subjected to physiological temperatures, e.g. temperatures of 34 to 40°C, more preferably of about 37°C, for a duration of at least 5 minutes, preferably for about 1 hour, but no longer than 2 hours.

Stripping remaining cell surface-bound binding agents from the cells in step (c1) of the method of the present invention is performed by incubating the cells with a suitable stripping buffer as known in the art. Such stripping buffers include acidic buffers, e.g. a buffer containing 0.5M NaCl and 0.5% (v/v) acetic acids, said buffer having a pH of 2.6. The duration of incubation is preferably 20 seconds to 1 minute, more preferably 30 to 40 seconds.

The secondary binding agent as used in steps (c1), (c2), and (c3) of the method of the present invention is not particularly limited, provided it is capable of binding to the first binding agent in a specific manner. Preferably, said binding agent is selected from the group consisting of antibodies, antibody fragments, and antibody mimetics, as defined above. Preferably, the secondary binding agent is an antibody, preferably a monoclonal antibody, directed against the first binding agent. In this context, the secondary binding agents employed in the above steps may be different from each other, or may be the same. Methods of intracellularly and surface labeling of cells are not particularly limited and are known in the art. They include steps of fixing cells, e.g. using paraformaldehyde, and then permeabilizing the cells, e.g. using saponin. The fluorescent label of the secondary binding agent and of the primary binding agent employed in step (c2)(ii)(α) is preferably a covalently bound fluorescent dye. Said fluorescent dye is not particularly limited, provided in can be detected in the method of the present invention. Suitable fluorescent dyes are known in the art and include for example Alexa Fluor® dyes (Invitrogen). In addition to the labeling of the internalized integrin of interest, surface integrin of interest and/or total integrin of interest, having the first binding agent bound thereto, additional cellular structures can be labeled by further fluorescent dyes. In a preferred embodiment, cell nuclei are stained, so that individual cells can be distinguished more easily.

In steps (c1)(v), (c2)(iii), and (c3)(iv) of the method of the present invention, the amount of fluorescent label in the intracellular compartment and/or the surface of the cells, as well as total fluorescent label, in each defined location is determined, preferably with the help of wide-field microscopy, confocal microscopy, total internal reflection fluorescence (TIRF) microscopy, or high-resolution microscopy (e.g., Stochastic Optical Reconstruction Microscopy (STORM)), either alone or in combination, preferably in an automated sequential and correlative manner. In particular, images are acquired and subsequently subjected to image analysis. During image analysis, individual total cells, the whole cell population, subpopulations of cells, or subregions of cells in a given defined location on the solid support can be analyzed.

In step (d) of the method of the present invention, the amount of the fluorescent label(s) determined in steps (c1) to (c3) for each defined location is compared to the amount of the fluorescent label(s) determined in the corresponding compartments of control cells in which no modulation of gene expression, no modulation of the activity of a gene product, no modulation of the expression of an ncRNA, and no application of a chemical agent has been performed. Alternatively, ratios of said amounts to each other for each defined location are compared to respective ratios determined in control cells in which no modulation of gene expression, no modulation of the activity of a gene product, no modulation of the expression of an ncRNA, and no application of a chemical agent has been performed. In this context, the terms "no modulation of gene expression", "no modulation of the activity of a gene product", and "no application of a chemical agent" are intended to encompass cells that have not been treated at all, as well as cells that have been treated with an inactive respective control agent, e.g. for experiments with protein-coding genes a control RNA that is not capable of down-regulating expression of any protein-coding gene, a control antibody, antibody fragment or antibody mimetic that is not directed against a relevant gene product, or a control chemical agent that is known to have no influence on integrin expression, localization and/or trafficking.

In step (e) of the method of the present invention, protein coding and protein non-coding genes, respective gene products, ncRNAs and/or chemical agents which modulate the expression, localization and/or trafficking of the integrin of interest are eventually identified, wherein such genes and/or gene products and/or ncRNAs and/or chemical agents are indicated by a statistically significant difference between the amount of the fluorescent label(s) determined in steps (c1)(v), (c2)(iii), and (c3)(iv) or respective ratios for each defined location, and the amount of the fluorescent label(s) or respective ratios determined in control cells in which no modulation of gene expression, no modulation of the activity of a gene product, no modulation of the expression of an ncRNA, and no application of a chemical agent has been performed. In particular, such statistically significant differences can be associated to particular functions of the modulated protein coding and protein non-coding gene and/or respective gene product, and/or the modulated ncRNA and/or the applied chemical agent.

In preferred embodiments, the method of the present invention further comprises the step of automatically classifying cells into discrete subpopulations according to their efficiency of endocytic trafficking of the integrin of interest after step (e), wherein said classification is obtained by using the Gaussian mixture model, assuming that in each subpopulation the cells are normally distributed. Respective statistical methods are known in the art. Furthermore, cells can be classified using any other relevant feature, like integrin expression level in individual cells (using combinatorial dual-label measurements). Any morphological cellular feature can be used to define cell populations (e.g., distribution of focal adhesions, endosomes). Cell population-based features can be used (e.g., cell density). Different cell- and population-based features can be combined in one read-out.

In particular embodiments, the method of the present invention further comprises the step of providing at least one additional stimulus to the cells at any point after or simultaneously with step (a) and prior to steps (c1) to (c3). This additional stimulus can be any stimulus of interest that is suspected to have an influence on the expression, localization and/or trafficking of integrins (e.g. epidermal growth factor (EGF) and/or fibroblast growth factor (FGF)).

In a particularly preferred embodiment, the method of the present invention is an automated method, in particular a fully automated method. In these embodiments, all methods steps as defined above are performed in an automated manner. Means for automating said method steps are not particularly limited and are known in the art. They include the deployment of liquid handling robots, automated sample and cell handling, automated image acquisition, automated image analysis, automated statistics, and automated data management, processing and analysis, including bioinformatics. Further, automatic contact printers can be used to coat particular substances to a solid support and to deliver living cells.

In preferred embodiments of the method of the present invention, the method steps are performed in the sequential order (a), then (b1) and/or (b2) and/or (b3) and/or (b4), then (c1) and/or (c2) and/or (c3), then (d), then (e). However, the steps (b1), (b2), (b3) and (b4), in case more than one of said steps is present, as well as the steps (c1), (c2), and (c3), in case more than one of said steps is present, can be performed in any particular order. Further, in certain embodiments, the compounds/agents for performing any of the steps (b1), (b2), (b3), and (b4) can be present on the solid support prior to the addition of cells to the solid support, so that said steps are performed as soon as said cells are added to the solid support, *i.e.* prior to or simultaneously with step (a). Furthermore, in certain embodiments, the cells can be contacted with the primary binding agent prior to adding said cells to the solid support, *i.e.* prior to steps (a) and/or (b1)/(b2)/(b3)/(b4).

The term "about" as used herein is intended to designate a variation of the respective value of -10% to +10%. As an example, the term "about 10" would be intended to encompass the range of "9 to 11".

The figures show:

### Figure 1:

### Endocytic trafficking of endogenous α2 integrins in HeLa cells.

Internalized fraction of the integrin α2 localizes to the juxtanuclear region and cytoplasmic structures after 1 h of internalization and to cytoplasmic structures after 6h of internalization (A). A large fraction of the integrin α2 remains on the plasma membrane during the same time of incubation (B). Antibody-clustered α2 integrin redistributes to the cytoplasmic structures (C). Quantification of the internalized α2 integrin (D) and integrin α2 on the cell surface (E). The amount of the internalized α2 integrin at the time point 1 h and surface integrin α2 at the time point 0h was set to 100% (Y axis) in (D) and (E), respectively. Bars represent average intensity of the internalized α2 integrin and error bars represent standard error of the mean from three independent experiments. Endocytic trafficking of integrin α2 is clathrin and caveolin dependent in HeLa cells (F, G). Cells were transfected with the control and test siRNAs, incubated for 48h, and integrin α2 internalization assay was performed. Depletion of clathrin heavy chain (CLTC), dynamin-2 (DNM2) and caveolin-1 (CAV1) efficiently inhibited integrin α2 internalization. Scale bar in (A,B,C,F) = 20 µm. Bars in (G) represent average amounts of intracellular integrin α2 after 1 h of internalization and scale bars represent standard error of the mean derived from three independent experiments. Amount of intracellular integrin α2 in cells transfected with the negative control was assigned to 100%. * p < 0,05, ** p < 0,01, *** p < 0,001.

### Figure 2:

### Internalized integrin α2 is re-cycled to the plasma membrane, but not degraded in HeLa cells.

Endogenous integrin α2 (red) was co-localized to the over-expressed GFP-tagged Rab5a (A), mCherry-tagged Rab11 b (B), GFP-tagged Rab4a (C), and GFP-tagged Rab7a (D) (green) 1 h after the internalization. Thick arrows show co-localizing structures and arrowheads show not co-localizing structures in overlay images. Thin arrows show perinuclear localization of overexpressed Rabs. Scale bar = 10 µm and scale bar of inserts = 5 µm.

### Figure 3:

### Fluorescent microscopy-based screen identifies potential regulators of integrin α2 endocytic trafficking.

(A) Distribution of z-scores of 386 tested genes in the primary screen (z-score value of the most effective siRNA is shown for every gene. Weak hits (z-score < +/- 1.5) are shown in mid gray, strong hits (z-scores > +/- 1,5) are shown in dark gray and listed, and non-effectors (z-score < +/-1) are shown in light gray. (B) Examples of integrin α2 endocytic trafficking assay on cell arrays. Down-regulation of dynamin-2 (middle column) and KIF18a (right column) induced a strong inhibition of integrin α2 intracellular accumulation. Intracellular integrin α2 specific fluorescence was measured within spot boundaries indicated by white lines on the images. Scale bar = 100 µm. (C) Comparison of potential regulators of integrin α2 endocytic trafficking to the regulators of transferrin and EGF endocytosis (left graph) and regulators of focal adhesion formation and epithelial cell migration (right graph).

### Figure 4:

### Validation of KIF15 role in integrin α2 endocytic trafficking.

(A) Depletion of KIF15 by RNAi prevents appearance of intracellular integrin α2 and this effect is lessened by the over-expression of GFP-tagged KIF15, but not GFP. Asterisks indicate cells expressing high levels of GFP and GFP-tagged KIF15. Scale bar = 20 µm. (B) Quantification of rescue of integrin α2 trafficking inhibition. Intracellular integrin α2 specific fluorescence is normalized to 100% when cells over-expressing GFP are treated with the negative control (Y axis). (C) Biotin-capture ELISA based assay shows that integrin α2 intracellular accumulation is inhibited when KIF15 is down-regulated. (D) Quenching antibody based assay shows that depletion of KIF15 inhibits integrin β1 intracellular accumulation. * p≤0,05, ** p≤0,01, *** p≤0,001. Down-regulation of KIF15 does not have influence on microtubules (E) and actin (F).

### Figure 5:

### KIF15 is specifically required for the internalization of integrin α2.

Down-regulation of KIF15 inhibits internalization of integrin α2 (A), but internalization of transferrin (B) and EGF (C) is not affected. In difference, re-cycling of transferrin to the plasma membrane is strongly inhibited (B). Scale bar = 20 µm. Quantification of integrin α2 (D), transferrin (E) and EGF (F) endocytic trafficking. Lines represent averaged intracellular intensities of each cargo, normalized to negative controls after 60 min of internalization of integrin α2 and 15 min of internalization of transferrin and EGF. Error bars indicate s.e.m. obtained from three independent experiments. * p≤0,05.

### Figure 6:

### Depletion of some CLASPs inhibits integrin α2 intracellular accumulation.

Down-regulation of Dab2 and ARH for 48h prevents integrin α2 intracellular accumulation (A, B), whereas down-regulation of Numb has no effect (B). (C) Down-regulation efficiency of Dab2 as shown by Western Blot. (D) Down-regulation of mRNAs encoding Dab2, Numb and ARH as tested by qRT-PCR. Scale bar = 20 µm in (A). * p≤0,05, ** p≤0,01.

### Figure 7:

### Empirical cumulative distribution function of intracellular α2-integrin.

α2-integrin specific fluorescence was quantified over the whole population of HeLa cells treated with the negative control. In a given specimen for 60% of the population (horizontal line) low fluorescence levels (vertical line) were observed.

### Figure 8:

### KIF15 potential influence on total and surface α2-integrin expression.

Down-regulation of KIF15 induces reduction of intracellular α2-integrin after 48h of incubation (A). Total level of α2-integrin expression is not changed when KIF15 is depleted, whereas, it is reduced when ITGB1 is down-regulated for 48h (B). Scale bar = 20 µm. Down-regulation of KIF15 increases α2-integrin surface expression as demonstrated by biotin-capture ELISA based assay (C). *p≤0,05, ** p≤0,01.

The present invention will be further illustrated in the following examples without being limited thereto.

### Examples

### Experimental procedures:

### Cell culture.

HeLa cells ATCC (CCL-2) were cultured in MEM (Sigma-Aldrich) containing 10% FCS, 2 mM glutamine, 100 U/ml penicillin and 100 µg/ml streptomycin. For the experiments requiring serum starvation, cells were kept in MEM with 0.01 % (w/v) BSA (MEM-BSA).

### Materials.

GFP-Rab5, GFP-Rab7a, mCherry-Rab11b, and mCherry-Rab4a were a gift of Dr. N. Brady (DKFZ/ BioQuant, University Heidelberg). GFP-MmKIF15 plasmid and anti-KIF15 antibody were a gift from R. Medema (The Netherlands Cancer Institute), mouse monoclonal anti-α2 integrin antibody (clone P1 E6) was from Merck-Millipore, rabbit monoclonal anti-Dab2 (H-110) and rabbit polyclonal anti-caveolin-1 were from Santa Cruz Biotechnology, mouse monoclonal anti-Dab2 (clone 52/p96) was from BD Biosciences, mouse monoclonal anti-clathrin and rabbit polyclonal anti-dynamin-2 were from Abcam, mouse monoclonal anti-α-tubulin (clone DM1A9) was from Cell Signaling, secondary anti-mouse and anti-rabbit antibodies coupled to Alexa-488 and Alexa-647 were from Invitrogen, anti-rabbit antibodies coupled to HRP were from GE Healthcare and anti-mouse antibodies coupled to HRP were from R&D. Transferrin-Alexa568, EGF-Alexa555 and phalloidin-Alexa647 conjugates were from Invitrogen. siRNAs targeting Dab2 (#SI02780316 and #SI02780386), ARH (#SI03042634 and #S100111657) and Numb (#SI04256994 and #S103199581) were from Qiagen.

### Transfection of siRNAs and cDNAs.

For the reverse transfection on cell arrays 5 µl of 30 µM siRNA was mixed with 3.5 µl Lipofectamine 2000 (Invitrogen) and 3 µl Opti-MEM (Invitrogen) containing 0.4 M sucrose and incubated for 20 min at RT. Thereafter, it was mixed with 7.25 µl of 0.2% gelatin (w/v) in 0.01 % fibronectin (v/v), and transferred to the contact printing with the automated liquid handling robot 'Microlab Star' (Hamilton) on 1-chamber Lab-Tek (Nunc) using eight solid pins PTS 600, which gives the spot size of approx. 400 µm. The spot-to-spot distance was set to 1125 µm, thus a single Lab-Tek fitted 384 spots organized in 12 columns and 32 rows. The whole library of 1084 siRNAs was spotted on 4 Lab-Teks, with 6-12 negative control siRNAs distributed randomly across each layout. 5x10⁵ cells were seeded/ per Lab-Tek. Liquid-phase direct transfection with siRNAs or cDNAs in 8-well µ-slide (Ibidi) was performed using Lipofectamine 2000 according to manufacturer's protocol with 2x10⁴ cells seeded/ per well. Transfection of cells with siRNA and cDNAs was done 48h and 24h before the assay, respectively.

### Western Blotting.

1.5x10⁵ HeLa cells were plated in 12-well plate, transfected with the respective siRNAs and cDNAs, lyzed in 80 ml hot Laemmli buffer, supplied with 100 mM DTT, and separated by 8 % SDS-PAGE. The proteins were transferred to PVDF membrane (Immobilion-P, Merck-Millipore), unspecific proteins were blocked with 5% milk in PBST, and incubation with the anti-KIF15 and anti-Dab2 antibodies was done at 4°C and RT for 1h, respectively. The proteins of interest were detected by ECL system (GE Healthcare). Images were recorded by Chemiluminescence Detection System (Intas) and quantified by Image J.

### qRT-PCR and RT-PCR.

Preparation of total cellular RNA was made using TRIzol reagent (Invitrogen) according to the manufacturer's protocol. cDNA was prepared with MMVL-reverse transcriptase (Ambion) and oligo-dT primer. Power SYBR Green PCR-Mastermix was used for the reaction, which was carried out in StepOnePlus Real-Time PCR system (Applied Biosystems). Relative Dab2, ARH and Numb expression after RNAi was calculated by 2^{ΔΔCT} method using expression level of GAPDH as a reference for the quantification. Expression of genes of interest in HeLa cells was tested by RT-PCR with 1-3 sets of primers that were determined by nucleotide BLAST analysis against human genome and transcriptome (http://blast.ncbi.nlm.nih.gov). Primers were either designed using Primer BLAST (http://blast.ncbi.nlm.nih.gov/tools/primer-blast/) or used as known in the art. Primers for all experiments were ordered from Metabion.

### Fluorescence microscopy based integrin α2 internalization assay.

Cells were serum-starved for 14h, overlaid with the 10 µg/ml anti-integrin α2 antibody in MEM-BSA for 1 h on ice, then shortly washed twice with ice-cold MEM-BSA to remove the excess of the antibody, and incubated with pre-warmed MEM-BSA at 37°C to internalize non-clustered integrin α2. After the internalization, cells were rinsed with PBS, surface bound antibody was removed with acetic buffer (0.5% (v/v) acetic acid, 0.5M NaCl, pH 2.6) for 30-40 s, and fixed with 2% PFA for 20 min at RT. Cells were permeabilized with 0.2% (w/v) saponin in 10% (v/v) FCS in PBS and incubated with the secondary antibodies for 1 h at RT. While measuring total integrin α2 levels, the treatment with the acetic buffer was omitted, cells permeabilized with 0.2% (w/v) saponin in 10% (v/v) FCS in PBS and stained with the primary and secondary antibodies. Integrin α2 on cell surface was measured by fixing cells with 2% PFA for 20 min at RT, quenching with 30 mM glycin for 5 min at RT and staining with the primary and secondary antibodies. Clustering by antibody was induced as described in the art by sequential incubation of cells with the primary anti-integrin α2 and then with secondary antibodies on ice for 1 h each. Then cells were shortly washed twice with ice-cold MEM-BSA and incubated with the pre-warmed MEM-BSA at 37°C to internalize antibody-clustered integrin α2. Nuclei were stained with 0.1 µg/ml Hoechst 33342 dye. In order to estimate the efficiency of acid stripping, the average values of total integrin α2 specific fluorescence, surface localized integrin α2 and intracellular non-clustered integrin α2 following 60 min of internalization using the same parameters for imaging and image analysis were compared. As expected, the highest average fluorescence intensity was obtained for total integrin α2 (100%) as it comprises intracellular and surface pools. Efficiency of acid stripping was considered as high, because the levels of non-clustered intracellular integrin α2 obtained after acid stripping (19.2%) and surface integrin α2 specific fluorescence (69,5%) makes up to 88,7% of the total integrin α2 fluorescence.

### Surface biotinylation based integrin α2 internalization assay.

The internalization of biotinylated integrin α2 was measured essentially as described in the art. Briefly, HeLa cells were overlaid with 0.2 mg/ml NHS-SS-biotin (Pierce) in PBS on ice for 30 min, washed with ice-cold PBS, and incubated in pre-warmed MEM-BSA at 37°C for required times. The cells were treated with 20mM MesNa (Sigma) in 50mM Tris (pH 8.6) in 100 mM NaCl on ice for 15 min to remove biotin remaining on cell surface. The reaction was quenched by addition of 20 mM iodoacetamide (Sigma) for 10 min. The cells were lyzed in 200 mM NaCl, 75 mM Tris, 15 mM NaF, 1.5 mM Na₃VO₄, 7.5 mM EDTA, 7.5 mM EGTA, 1.5% Triton X-100, 0.75% Igepal CA-630 and proteinase inhibitor cocktail (Roche). Lysates were passed three times though a 27G needle and centrifuged for 10 min at 10000 x g. Amount of biotinylated integrin was determined by capture-ELISA, which was performed as described in the art.

### Quenching antibody-based integrin β1 internalization assay.

The assay was essentially performed as described in the art. Briefly, surface integrin β1 was labeled 1 h on ice using CD29 clone K20 antibodies (Beckman Coulter) conjugated to AlexaFluor 488. Integrin β1 was endocytosed for 1 h at 37°C and remaining cell surface fluorescence was quenched using anti-AlexaFluor488 antibody (Invitrogen). After cell fixation, fluorescence intensities were measured by EnVision Multilabel Plate Reader (Perkin Elmer).

### EGF and transferrin internalization assays.

Cells were serum-starved for 14 h before the assay; then EGF-Alexa555 was added to MEM-BSA medium to a final concentration of 100 ng/ml and cells were incubated at 37°C for different time points. Surface attached EGF-Alexa555 was removed with acidic buffer (50 mM glycine, 150 mM NaCl, pH 3.0) and cells were fixed with 3% PFA for 20 min at RT. For transferrin endocytic trafficking assay cells were serum-starved for 1 h, then tranferrin-Alexa568 was added to MEM-BSA medium to a final concentration of 25 µg/ml. Cells were incubated at 37°C for different time points and fixed with 3% PFA for 20 min at RT.

### Image acquisition by wide-field, confocal and TIRF microscopy.

Image acquisition in wide-field mode was done on Olympus IX81 Scan^R automated inverted microscope (Olympus Biosystems) controlled by Scan^R acquisition software. 10x/0.4 NA air objective (UPlanSApo; Olympus Biosystems) was used for the primary screening on cell arrays and 20x/0.75 NA air objective (UPlanSApo; Olympus Biosystems) was used for all other experiments. Excitation wavelength = 450-490 nm and emission wavelength = 500-550 nm was used to image integrin α2, excitation wavelength = 426-446 nm, emission wavelength = 460-500 nm - to image EGF-Alexa555, and 545-580 nm excitation and 610-700 emission wavelength - to image transferrin-Alexa568. Excitation wavelength = 325-375 nm, emission wavelength = 435-475 nm was used to image nuclei in all assays. Confocal imaging was performed on a confocal laser scanning microscope TCS SP5 (Leica Microsystems) using a 63x oil immersion objective (HCX PL APO 63x/1.4-0.6 Oil CS) and 94 µm diameter pinhole. 488, 561 and 633 nm laser lines were used for the excitation of GFP or Alexa488, mCherry or Alexa568, and Alexa 647, respectively. TIRF was performed on Nikon Eclipse Ti TIRF microscope with Nikon Apo TIFR 60x NA 1.49 objective. 488 and 640 nm laser lines were used for excitation of Alexa488 and Alexa647, respectively.

### Co-localization analysis.

Co-localization of internalized integrin α2 with overexpressed Rabs in the multi-channeled single confocal plane images was done in Image J. For this, the image background was subtracted using a rolling ball algorithm, noise was removed by Gaussian Blur filter and individual structures of size more than 10 pixels were binarized and overlapped. In total, peripheral regions of more than 5 cells/ Rab were used for analysis with more than 150 structures analyzed per cell.

### Statistical data analysis.

During the primary screening, one image completely encompassed an individual spot. Prior to quantification of the internalized integrin-specific fluorescence intensity, background signal was subtracted applying a rolling ball algorithm implemented in the Scan^R Analysis module (Olympus). All images were subject to visual control to exclude those of insufficient quality. The nuclei of individual cells were identified by intensity module and the region of nuclei was expanded so as to encompass a maximum area of cell without touching the neighboring ones. Cell densities were optimized to reach 60-90% confluence on a spot to secure clear cell-to-cell separation. As the majority of intracellular integrin α2 specific fluorescence was clustered in the region of interest, the method allowed unbiased data quantification. For the statistical analysis of the primary screening data of integrin α2 internalization R package (http://cran.r-project.org/) and cellHTS from Bioconductor (http://www.bioconductor.org/) were used. At first, 2% of the cells with the highest integrin-specific fluorescence intensities were excluded for each imaged spot, remaining cell intensities were averaged, and B-score normalization (Brideau et al., 2003) was applied to calculate a correction factor for each spot, which would account for spatial effects and between-plate artifacts.

To reliably quantify integrin α2 endocytic trafficking, attention was focused only on cells which show an efficient endocytic trafficking. The threshold to remove cells with weak endocytic trafficking was set for every experiment separately according to the negative controls and kept the same for all samples within the experiment. Gaussian Mixture Model was used to automatically cluster the cells into two subpopulations (with high and low efficiencies of endocytic trafficking of integrin α2) assuming that in each subpopulation the cells are normally distributed. The median intracellular integrin α2 signal intensities and cell numbers of each subpopulation was calculated for every spot. Then, the median signal intensity of cells with efficient endocytosis was multiplied to the number of such cells and divided by the total cell numbers in every spot. This ratio was normalized against the median of the negative controls on each cell array. Eventually, median z-score of 5-8 individual replicates of every siRNA was calculated and one-sided, one-sample Welch's t-test was used to compute significance values. A gene was considered as a primary hit when either one out of two tested siRNAs, at least two siRNAs out of six tested, or at least three siRNAs out of more than eight siRNAs tested had a consistent inhibitory or acceleratory effect on integrin α2 intracellular accumulation. More than 3000 cells were analyzed for every siRNA in the primary screen.

For the validation experiments in 8-chamber µ-slides, 30 to 42 images/ well were taken and analyzed. All other steps of the analysis were the same as in the primary screening, except that a threshold, separating cells with high and low endocytosis, was set manually. For transferrin and EGF trafficking assays all cells in population were taken for the analysis.

For analysis of the rescue experiment, individual cells were divided into subpopulations according to fluorescence intensity of the over-expressed GFP and GFP-KIF15. Integrin α2 specific fluorescence in cells with the highest 30% of GFP-KIF15 intensity were averaged and normalized to that of all cells expressing GFP. Statistical significance of difference between experiments throughout this study was tested by Student's two-tailed t-test for samples with uneven variance.

### Example 1:

### Endocytic trafficking of integrin α2.

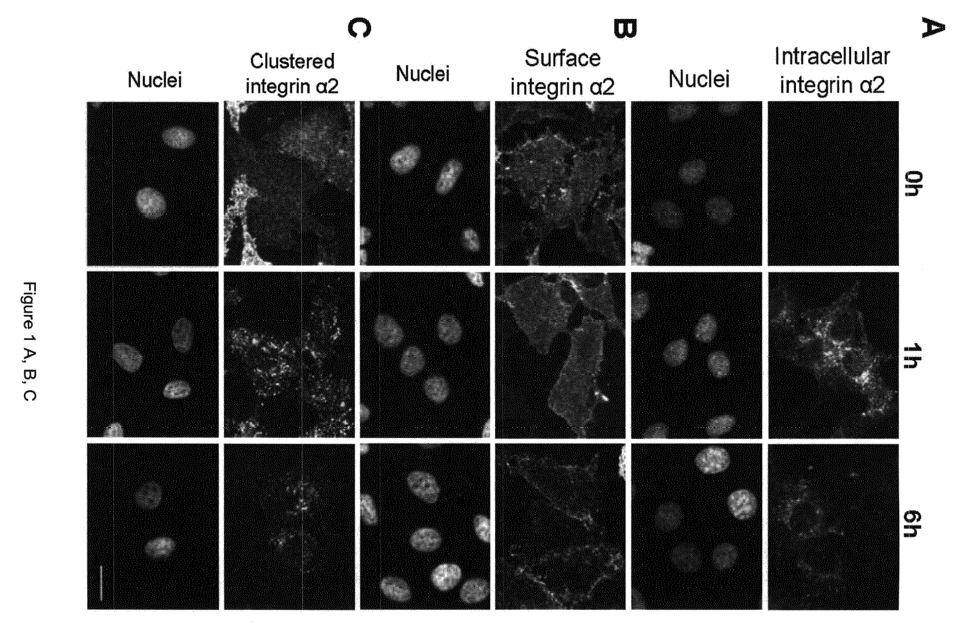

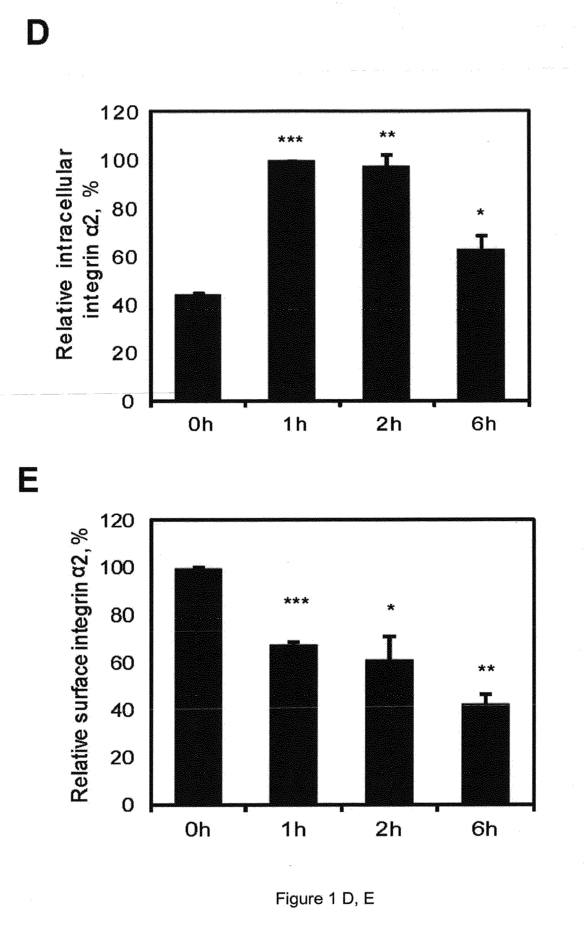

It was initially tested whether endocytic trafficking of integrin α2 is similar or different from what is known about integrin β1 in HeLa cells. Integrin α2 was labeled with the antibodies at 4°C for 1 h and then internalized at 37°C at varying time points in HeLa cells. In order to quantify the amount of the intracellular integrin α2, the integrin-bound antibodies remaining on the cell surface were removed by treating cells with the acidic buffer. Internalized integrin α2 accumulated at the juxtanuclear area and cytoplasmic structures (Fig. 1A). Intracellular integrin α2 specific signal increased two-fold after 1h of incubation (Fig. 1 D). Integrin α2 localized largely to the cytoplasmic structures after prolonged incubation time of 6h (Fig. 1A). Progressive reduction of 35%, 48%, and 60% of the cell surface integrin α2 was measured by immunofluorescence staining after 1 h, 2h and 6h of incubation, respectively (Fig. 1B,E). Similarly, the surface integrin β1 in HeLa cells was diminished by 50% within 30 min of incubation and this level did not significantly change up to 2h of incubation.

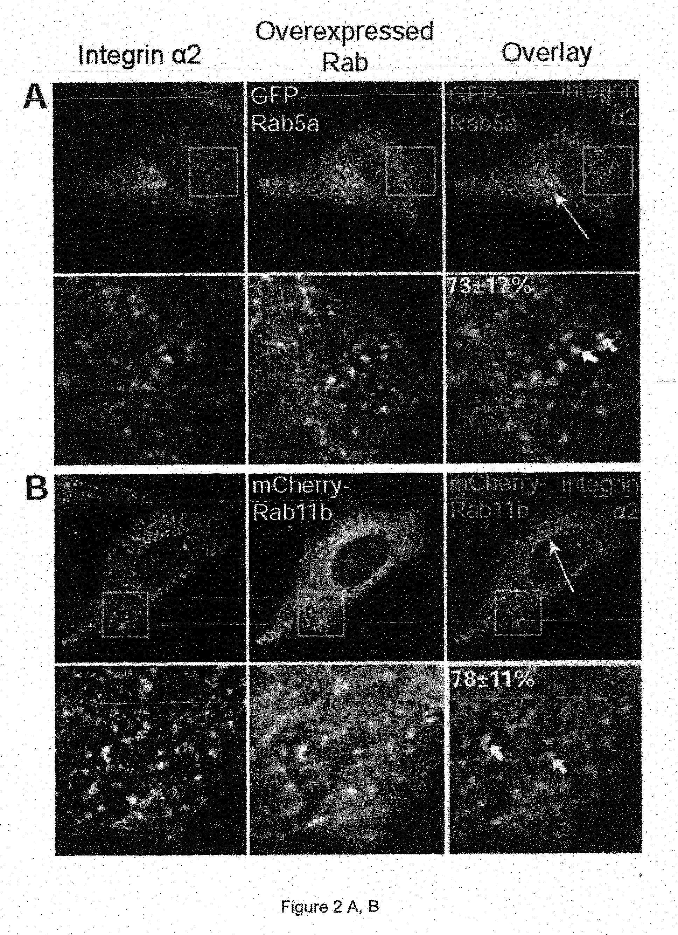

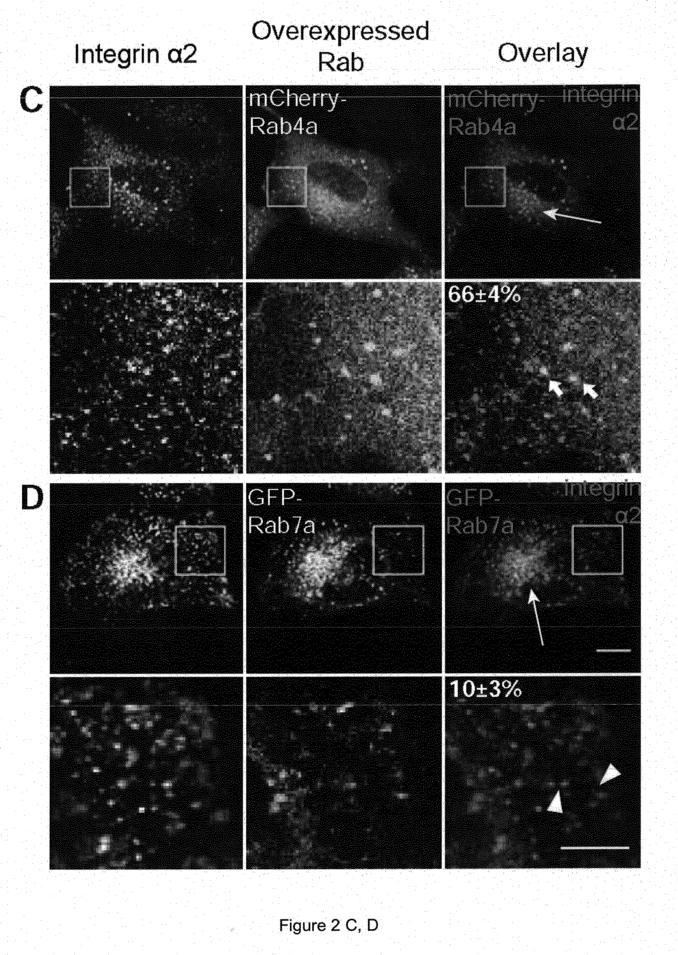

Similar to integrin β1, more than 70% of integrin α2 co-localized to the peripheral structures specific for over-expressed Rab5a GTPase following 1 h of internalization (Fig. 2A). Only 10% of the peripheral integrin α2 containing structures were Rab7a positive (Fig. 2D), which is similar to what was reported for inactive integrin β1. In addition, the pattern of perinuclear localized integrin α2 strongly resembled that of Rab5a (Fig. 2A), but was different from the distribution of Rab7a (Fig. 2D). Presumably, integrin α2 enters the lysosomal degradative pathway inefficiently. In contrast, around 70% of the peripheral integrin α2 containing structures were Rab11b and Rab4a positive (Fig. 2B,C), which indicates that integrin α2 enters short- and long-loop re-cycling pathways. Interestingly, perinuclear localization of integrin α2 was reduced when Rab11b and Rab4a were overexpressed (Fig. 2B,C); no such observation was reported for the internalized integrin β1 under similar conditions.

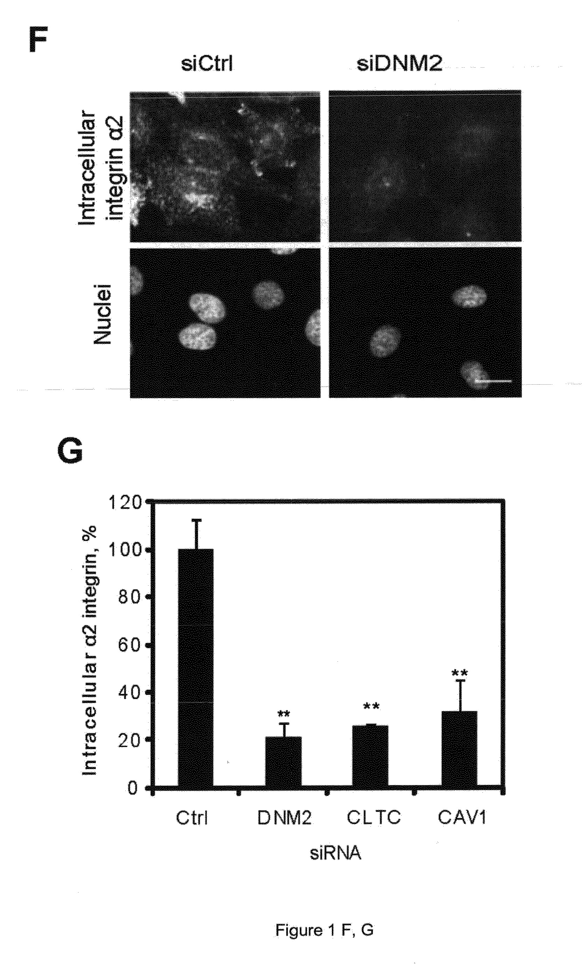

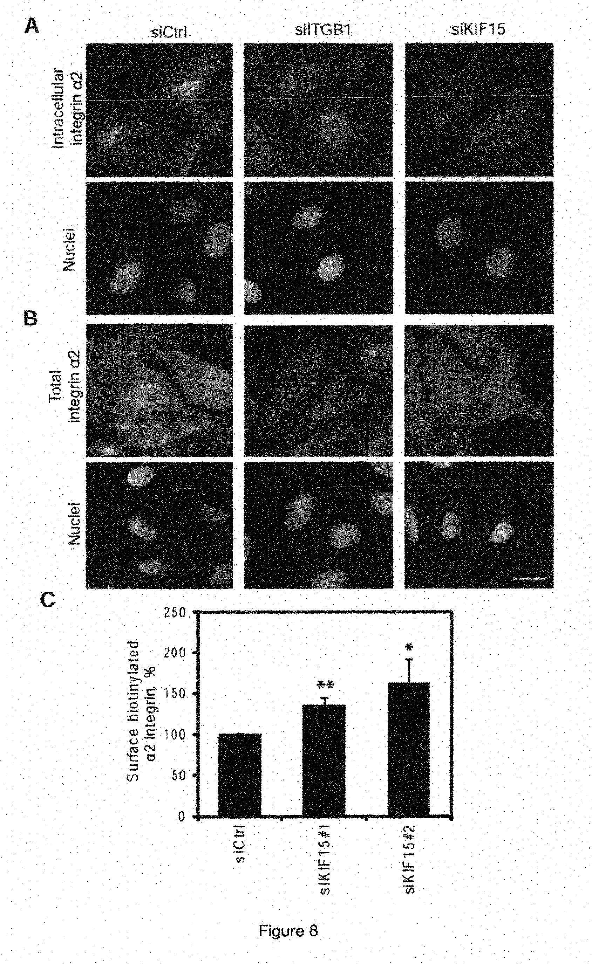

RNAi experiments showed that endocytosis of integrin β1 occurs via clathrin-dependent pathway in various types of cells and caveolin-dependent pathway in myofibroblasts. The latter is also utilized by integrin α2 in ovarian carcinoma cells when EGFR is activated or upon clustering of integrin α2β1 by antibodies. As these observations might be cell-type and integrin-dependent, it was tested whether both of these pathways play a role in endocytic trafficking of integrin α2 in HeLa cells. For this, the key components of clathrin-dependent and caveolae-dependent pathways (clathrin heavy chain (CLTC), caveolin-1 (CAV1), and dynamin-2 (DNM2)) were depleted using validated siRNAs. Prior to internalization assays, the efficiency of siRNAs was tested by Western Blotting or immunofluorescence staining, and around 50% of the target proteins were depleted after 48h of incubation (Fig. 8). Even being not complete, the down-regulation of CLTC, dynamin-2 and caveolin-1 inhibited internalization of integrin α2 by 70-80% (Fig. 1F,G).

According to these data, endocytic trafficking of integrin α2 in HeLa cells is similar to integrin β1, mostly to its inactive form. In contrast, little features are shared with the trafficking of antibody-clustered integrin α2. For instance, clustered integrin α2 enters calpain-dependent degradative endosomal route and is not re-cycled back to the plasma membrane in SAOS cells over-expressing α2 integrin. By applying the similar protocol to the endogenous integrin α2 in HeLa cells, accumulation of α2 in cytoplasmic punctate structures was observed with hardly any plasma membrane localization at different time points (Fig. 1 C).

### Example 2:

### Potential regulator of integrin α2 endocytic trafficking identified by RNAi assays.

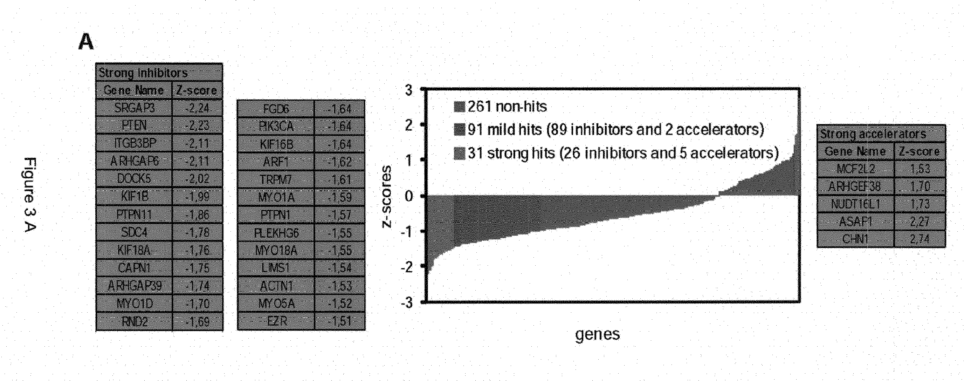

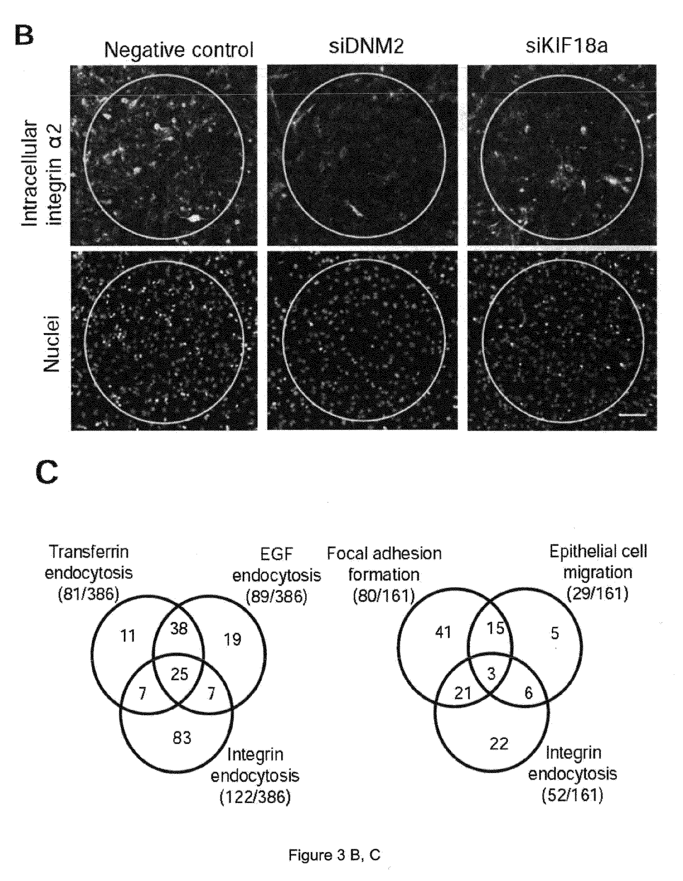

To identify regulators of endocytic trafficking of integrin α2, a fluorescent microscopy-based RNAi screen was performed with 1084 siRNAs targeting 386 genes. The selected library (provided by Dr. R. Pepperkok, EMBL) comprised 94 molecular motors (44 kinesins, 13 dynein subunits, and 37 myosins), 28 small Ras and Rho GTPases, 53 GAPs and 62 GEFs of these GTPases, 41 actin-associated and 14 microtubule-associated proteins, 34 kinases and 9 phosphatases, 5 different integrin β chains and 23 scaffold and adaptor proteins as well as a number of proteins belonging to diverse functional groups. For the primary screening, reverse transfection on cell arrays was used. All replicas of Lab-Tek chambers, which were used in the screen, with spotted 384 siRNAs were prepared in one experiment, allowing to ensure a high efficiency and reproducibility of the whole screen. Although cell arrays contain fibronectin and gelatin, no clustering of integrin α2 was observed under these conditions. Data were collected on an automated wide-field microscope and intracellular integrin α2-specific fluorescence was quantified for single cells after 1 h of internalization when the level of intracellular integrin α2 was the highest in the control specimen (Fig. 1C), thereby allowing the best signal-to noise ratio during the screen. The inspection of the control samples over large cell populations revealed that the amounts of internalized integrin α2 vary strongly in HeLa cells. As quantification of intracellular integrin α2 in cells with low efficiency of endocytosis is unreliable and could have a negative impact on screen results, attention was focused on cells with efficient endocytosis. For this, according to visual inspection, a threshold was set for cells with low levels of intracellular integrin α2 defined under the conditions of image acquisition. Then, the empirical cumulative distribution function was used to define the respective cell population for every experiment (Fig. 9) and Gaussian Mixture Model to separate cell subpopulations. Usually, low levels of intracellular internalized integrin α2 were found in up to 60% of HeLa cells transfected with the negative control and these were disregarded from the further analysis. Screen hits were defined according to individual z-scores of each siRNA normalized to z-score of the negative controls that were set to 0. Positive z-scores indicate increased amounts of intracellular α2 and negative z-scores indicate reduced amounts of intracellular α2. As the primary hits, siRNAs with z-scores > +/- 1 were considered. 122 genes were assigned as potential hits in the screen: 115 of them inhibited and 7 of them accelerated integrin α2 intracellular accumulation (Fig. 3A). Next, the primary hits were separated into strong (z-score > +/- 1.5) and weak effectors (z-score < +/- 1.5). Down-regulation of CLTC and dynamin-2 is expressed as z-score of -1,5 and corresponds to a strong inhibition of integrin α2 trafficking on cell arrays (Fig. 3B). 26 other genes were classified as strong inhibitors, some of them outperforming CLTC and dynamin-2 when down-regulated. For instance, down-regulation of syndecan-4, which triggers internalization of integrin via caveolae-dependent pathway, induced a strong inhibition (z-score = -1.78) of integrin α2 intracellular accumulation. The majority of inhibitors are mild (90 out of 115); even depletion of caveolin-1 for 48h showed reproducible, but weak inhibitory effect (z-score = -1,14). 7 primary hits of the screen were called "accelerators". One of them is ASAP1, a GAP of ARF6, previously shown to regulate integrin re-cycling. It was scored as a strong accelerator (z-score = 2.26); that effect caused by the enhanced accumulation of intracellular integrin α2, likely due to inhibited re-cycling.

Having classified the primary hits in functional classes according to the Gene Ontology terms, significant enrichment among strong effectors was not found, except for diverse kinases and phosphatases that were more frequently found among strong hits (19%) in comparison to the screened library (11 %). While comparing the primary hits to a genome-wide screening for regulators of transferrin and EGF endocytosis, it was found that 39 of 122 hits (32%) were reported as regulators of either transferrin or EGF endocytosis (Fig. 3C). 25 of them influenced trafficking of all three cargoes when down-regulated (e.g., ARHGAP6, a GAP of RhoA). Kinases and phosphatases were also over-represented in this pool of potential regulators (24%). 83 primary hits from the screen might be specific only to integrin α2 intracellular accumulation. No significant enrichment of any functional class could be identified, except kinases and phosphatases that were under-represented (6%).

As endocytic trafficking of integrins contributes to the dynamic of focal adhesions and cell, the results of the screen were compared to relevant RNAi screens known in the art (Fig. 3C). 161 genes from the above experiments were also screened to identify regulators of focal adhesions and cell migration. 24 out of 161 genes (15%) were identified as the primary hits for both integrin α2 trafficking and formation of focal adhesions, for instance, phosphatidylinositol phosphatase PTEN. 9 out of 161 genes (5.5 %) were positive in the above assays and cell migration assays.

A fairly high overlap of the data was also obtained when comparing to the components of integrin-primed complexes. 45 out of 467 identified non-redundant components of integrins α5β1 and α4β1 were tested in the screen and 19 of them were assigned as potential regulators of integrin α2 intracellular accumulation (e.g., molecular motors KIF2C and cytoplasmic dynein 1 heavy chain 1 DYNC1H1). A recent search for integrin β1 interacting proteins yielded 96 proteins, from which 11 were tested in the present study, and four of them were scored as the primary hits. Three of them, namely, integrin activating protein Kindlin-1, an adaptor protein LIMS1 that connects integrin, small GTPase and EGF signaling pathways and RALBP1, a GAP of RAC1 and CDC42 are closely related to integrin cellular functions. Besides known regulators of integrin function, the above screen revealed a number of novel potential regulators that were previously not associated to integrin trafficking. For instance, KIF18A, a key component of chromosome congression in mitosis, induced a strong inhibition of integrin α2 intracellular accumulation when down-regulated (Fig. 3B).

### Example 3:

### Validation of the potential regulators of integrin α2 intracellular accumulation.

About 1/5 of the primary hits (26) were initially selected for validation. One selected group comprised molecular motors kinesins as little is known about their roles in integrin endocytic trafficking and nearly all known kinesins have been tested in the primary screening. 15 kinesins were identified as potential inhibitors and the expression of 12 kinesins was tested (for kinesins having isoforms only one isoform was included) with 9 of them found to be expressed in HeLa cells. Different from the primary screening (cf. Fig. 3B), validation assays were performed in non-coated multi-well plates under conditions of a direct transfection. Intracellular accumulation of integrin α2 was considered as inhibited when intracellular amount of integrin α2 was reduced by more than 40% compared to the negative control. Potential hits were scored as validated when at least two out of four siRNAs used for the validation experiments showed the same effect in integrin α2 intracellular accumulation as in the primary screening. Three kinesins (KIF15, KIF18A, KIF23) were validated as effectors of integrin α2 intracellular accumulation (Table 1). As few kinesins, known trafficking regulators, were scored as hits in the primary screening, 17 traffic-related kinesins were included in the validation procedure. 13 of them are expressed in HeLa cells; and only KIF13A, that is known to regulate re-cycling in melanocytes, showed an effect on integrin α2 intracellular accumulation in the validation experiments. As this result was obtained only with the additional siRNAs, but not with the ones used for the primary screening, KIF13A was not included in the list of the validated hits.

**Table 1. Validated regulators of integrin α2 intracellular accumulation.**

| **Gene name** | **Intracellular integrin α2, %** | **Total integrin α2, %** | **Cell count, %** |
|---|---|---|---|
| Negative control | 100 | 100 | 100 |
| **DNM2** | 27,2 ± 6,63 | 82,45 ± 13,77 | 98,56 ± 6,88 |
| **ARF1** | 49,17 ± 7,43 | 99,11 ± 17,67 | 99,16 ± 37,85 |
| ABL2 | 52,78 ± 9,94 | 56,84 ± 4,49 | 72,05 ± 18,6 |
| ARHGAP6 | 42,03 ± 6,35 | 43,14 ± 4,33 | 75,59 ± 7,24 |
| ITGB1 | 7,38 ± 3,4 | 54,12 ± 7,1 | 99,93 ± 17,32 |
| **KIF15** | 27,8 ± 4,45 | 96,5 ± 2,3 | 106,44 ± 7,75 |
| **KIF18A** | 51,12 ± 9,54 | 97,64 ± 16,74 | 98,64 ± 31,44 |
| **KIF23** | 25,4 ± 6,36 | 92,69 ± 33,23 | 49,11 ± 22,74 |
| Myo1A | 36.43 ± 17.79 | 64,72 ± 4.04 | 126,42 ± 46,71 |
| **NF2** | 35,59 ± 3,1 | 95,89 ± 13,64 | 85,77 ± 3,19 |
| PTPN11 | 39,11 ± 5,19 | 76,83 ± 3,72 | 66,07 ± 28,1 |

Validated regulators that inhibit integrin α2 intracellular accumulation without changing integrin α2 expression are shown in bold. Intracellular and total levels of integrin α2 represents average values (± S.E.M) of integrin α2 specific fluorescence derived from cells showing efficient endocytosis and all cells, respectively.

Next, it was tested whether decreased levels of intracellular integrin α2 are a direct consequence of inhibited trafficking or reduced expression level of integrin α2. For this, total amount of integrin α2 was measured following down-regulation of the validated kinesins (Table 1). Expression levels of integrin α2 were not affected when KIF15, KIF18A and KIF23 were down-regulated (Fig. 10 A,B). As these kinesins were shown to play a role in mitosis, the number of cells remaining after 48h of transfection with the respective siRNAs was calculated. Indeed, down-regulation of KIF23 reduced the number of cells nearly two-fold, whereas down-regulation of KIF15 and KIF18A did not induce a significant change of cell numbers under these conditions (Table 1). To get a better overview about the specificity of the screen, additional 14 primary hits were included in the validation procedure. These are a functionally heterogeneous group of potential regulators that were scored as effectors in the primary screening with multiple siRNAs. Down-regulation of seven of them strongly reduced levels of the intracellular integrin α2 in the validation assays (Table 1). Five of them were also classified as strong inhibitors in the primary screen (ARF1, ABL2, ARHGAP6, Myo1A and PTPN11). Two out of seven validated regulators (ARF1 and NF2) did not change expression level of integrin α2, and therefore primarily may act on integrin trafficking. ITGB1 and Myo1A reduced expression of integrin α2 when down-regulated, but probably play a role also in the trafficking of this cargo as their inhibitory effect on its intracellular accumulation is stronger than on the expression level (Table 1). On the other hand, inhibitory effect measured after down-regulation of ABL2 and ARHGAP6 largely accounts for the reduction of the expression level. Cell numbers were reduced by nearly 35% when PTPN11 was down-regulated, whereas down-regulation of other molecules induced rather mild effect on cell numbers.

Thus, 10 out of 23 (43%) potential inhibitors of intracellular integrin α2 accumulation could be validated. Curiously, for nearly half of them this effect was primarily attributed to the reduced integrin α2 expression level. Remaining molecules might be directly involved in integrin α2 trafficking or may act indirectly by regulating, for instance, degradation.

### Example 4:

### Internalization of integrin α2 is KIF15 dependent.

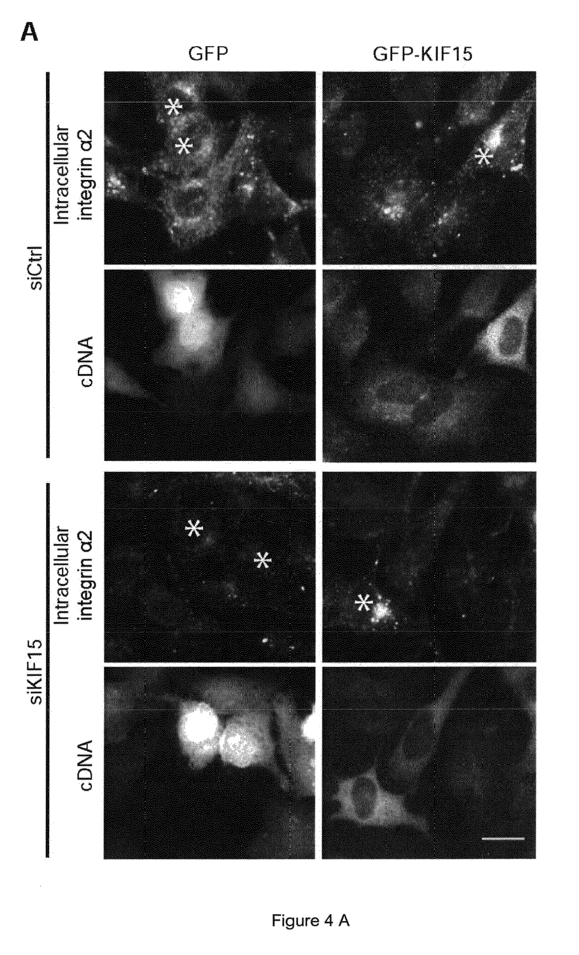

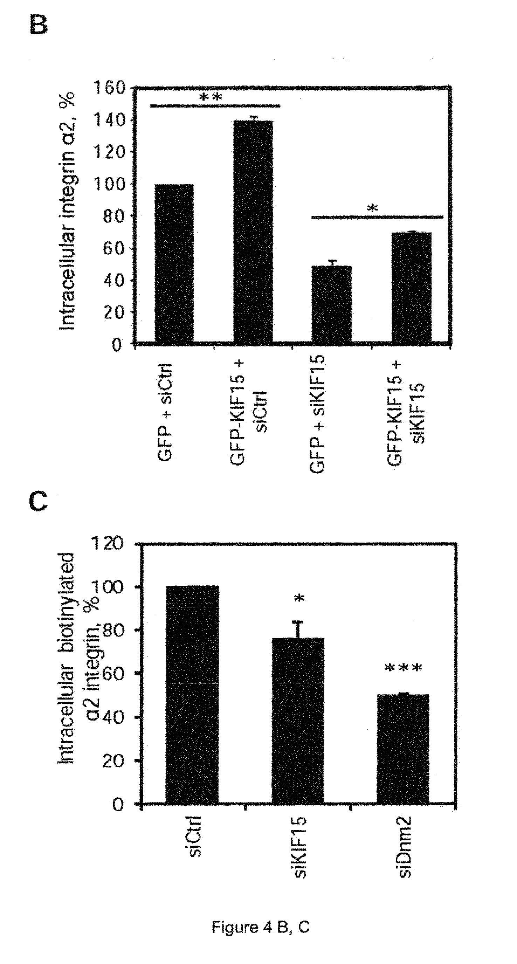

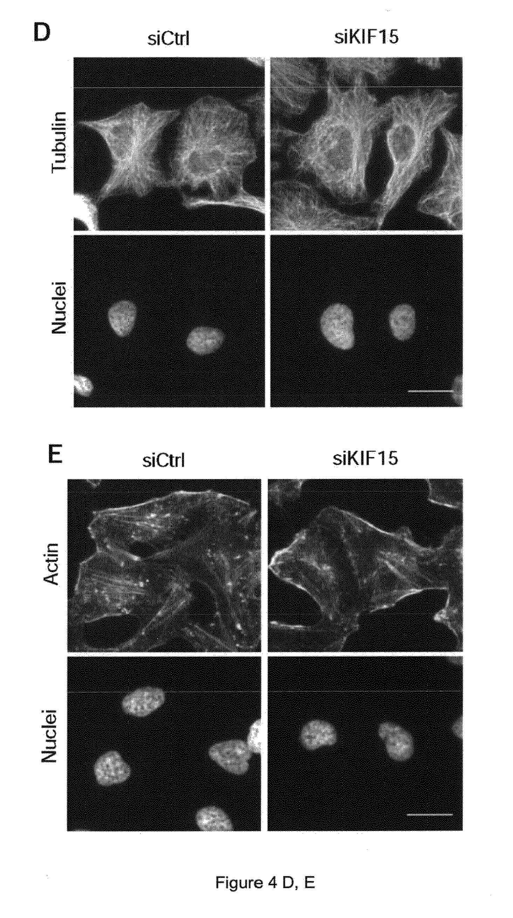

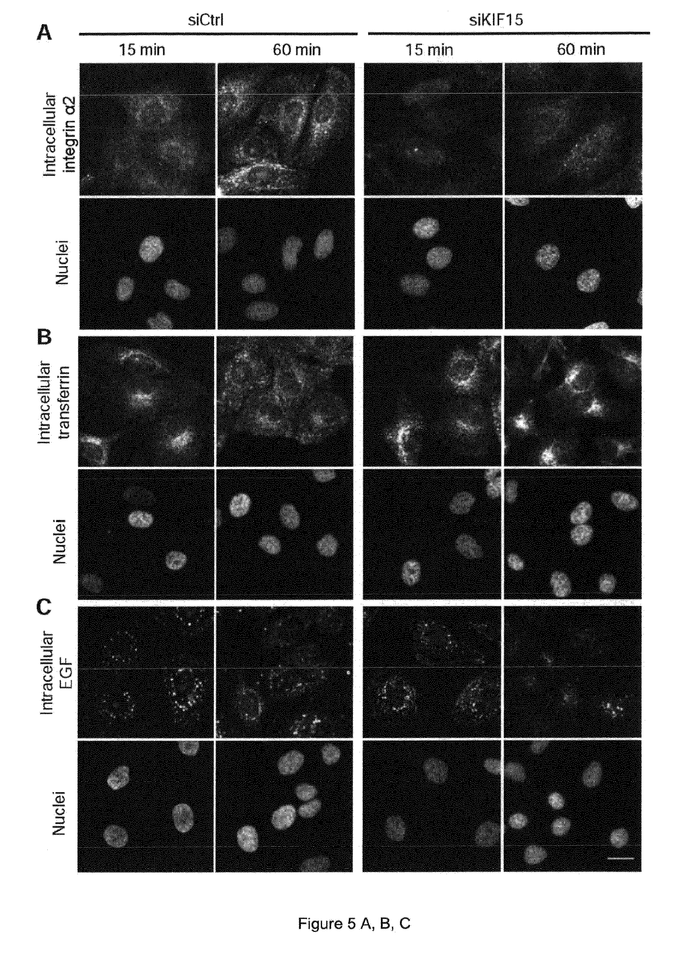

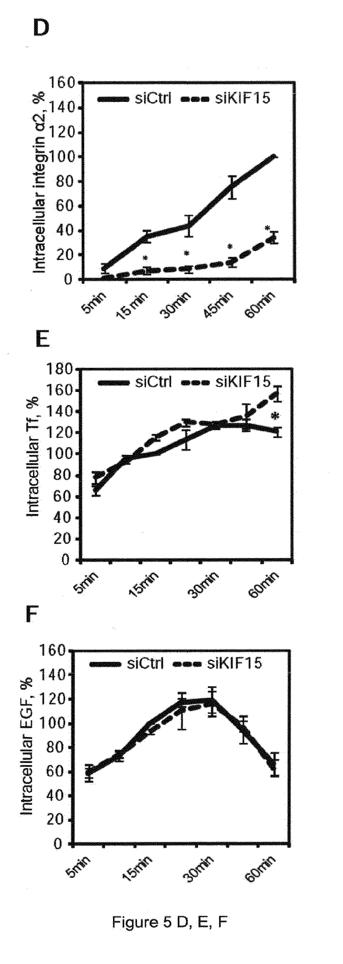

To understand the possible regulatory function of validated novel effectors in integrin α2 trafficking, attention was focused on one of the strongest effectors in a course of validation, namely KIF15, for further studies. This molecular motor strongly inhibited integrin α2 intracellular accumulation when down-regulated without significantly changing integrin α2 expression level and cell numbers in population (Table 1, Fig. 10 A, B). In addition, no trafficking-related function has been described for KIF15 so far. At first, it was tested by Western Blotting that more than 70% of KIF15 was depleted in HeLa cells following 48h after transfection (Fig. 11 A, C). Next, it was assayed whether over-expression of human siRNA-resistant murine KIF15 tagged to GFP rescued inhibition of integrin α2 intracellular accumulation. Moderate expression levels (3.3-fold) of the ectopic KIF15 compared to the endogenous protein were obtained in HeLa cells after 24h of transfection (Fig. 11 B, D). GFP-tagged KIF15 mostly localized to the cytoplasm (Fig. 4A) with occasional localization to the plasma membrane, punctate cytoplasmic structures and microtubules. Over-expression of KIF15 induced an increase of intracellular integrin α2 amounts nearly by 40% (Fig. 4A,B), with integrin α2 accumulating at the perinuclear region after 1 h of internalization. Over-expression of GFP under these conditions had no apparent influence. In contrast, a strong inhibition of integrin α2 intracellular accumulation was registered when KIF15 was down-regulated. The over-expression of GFP-tagged KIF15 lessened this effect by 30% (Fig. 4B). The role of KIF15 in integrin α2 endocytic trafficking was further confirmed by biotin-capture ELISA assay (Fig. 4C). Furthermore, endocytosis of integrin β1 was also inhibited as measured by ratiometric quenching antibody-based assay (Fig. 4D). Both assays revealed lower inhibition rate of integrin trafficking (around 25%) when KIF15 was down-regulated than the microscopy-based assay (Table 1). This can likely be attributed to the difference in quantifying the whole cell population in these assays and only a defined subpopulation of cells for the microscopy-based assay. Finally, no significant changes of actin and microtubule cytoskeleton was observed following KIF15 down-regulation (Fig. 4D,E). Next, it was analyzed at which stage integrin α2 intracellular accumulation is affected when KIF15 is depleted. In cells transfected with the negative control, integrin α2 was gradually accumulating, whereas down-regulation of KIF15 strongly inhibited intracellular accumulation of integrin α2 starting from early time points (Fig. 5A). The inhibition remained until longer incubation times (Fig. 5D), pointing out that internalization of integrin α2 may be perturbed under these conditions. In agreement, integrin α2 surface expression increased when KIF15 was down-regulated (Fig. 10 C). The effect of KIF15 down-regulation on the trafficking of EGF that is degraded in lysosomes and transferrin that is re-cycled to the plasma membrane was then tested. In agreement to findings known in the art, down-regulation of KIF15 had no influence on internalization of both cargoes at early time points (Fig. 5B,C,E,F). Yet, the later steps of transferrin trafficking were significantly affected by KIF15 down-regulation (Fig. 5E) with the cargo accumulated in the perinuclear region (Fig. 5B). Presumably, the observed effect is due to inhibited re-cycling of transferrin to the plasma membrane. In contrast, EGF endocytic trafficking was not influenced by KIF15 down-regulation at any time point (Fig. 5C,F).

### Example 5:

### KIF15 is required for cell surface localization of Dab2.

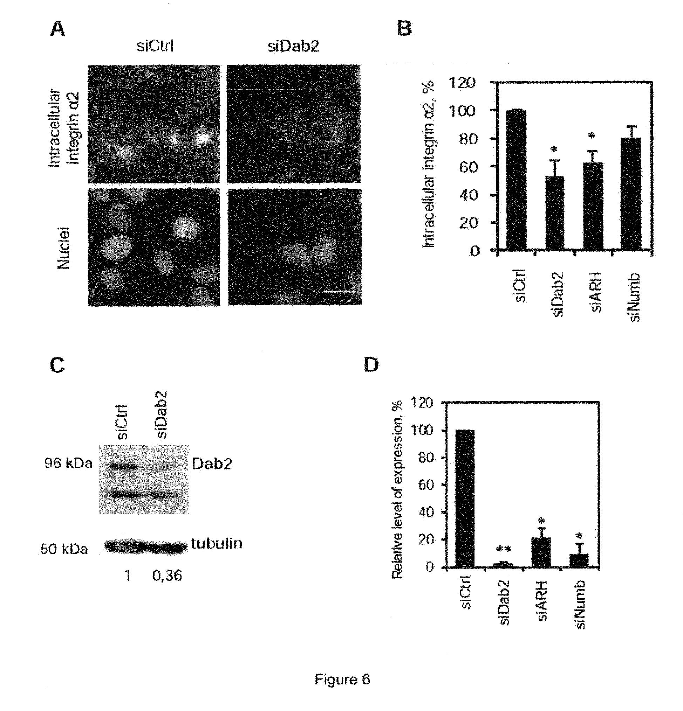

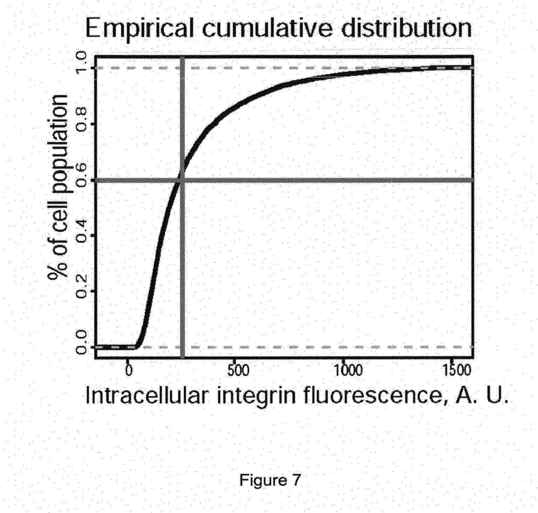

The observed cargo-specific difference was investigated further. It was shown that internalization of transferrin receptor is not dependent upon alternative clathrin adaptors CLASPs, like Dab2. On contrary, several CLASPs were shown to be required for integrin internalization. Dab2, ARH and Numb were down-regulated in HeLa cells for 48h and integrin α2 intracellular accumulation was measured (Fig. 6). The efficiency of down-regulation of the respective transcripts was more than 80% as tested by qRT-PCR (Fig. 6D). Reduction of Dab2 protein by more than 60% (Fig. 6C) induced the strongest inhibition of integrin α2 endocytic trafficking. Depletion of ARH had weaker effect, whereas depletion of Numb showed no significant inhibition (Fig. 6A,B). These data are in a good agreement with prior art studies, showing that Dab2 strongly inhibits internalization of integrin β1 when down-regulated. Furthermore, surface levels of integrins β1 and α2 increase in Dab2-deficient Hela cells. To explain the different effects of KIF15 on integrin α2 and transferrin internalization, it was analyzed whether the strongest effector of integrin α2 trafficking, Dab2, is functionally dependent upon KIF15. In agreement to previous studies, Dab2 localized to punctate structures that were found both on the plasma membrane and intracellularly in HeLa cells imaged in TIRF and epifluorescence modes, respectively (Fig. 7A). Down-regulation of KIF15 for 48h induced more than 40% loss of the cell surface Dab2 (Fig. 7C, D) with a large fraction of Dab2-specific punctate structures redistributed from the plasma membrane to the cytoplasm (Fig. 7B). Total level of Dab2 in cells was not changed significantly as measured by Dab2-specific fluorescence (Fig. 7B) and Western Blotting (Fig. 4F). Surprisingly, no significant co-localization between Dab2 and integrin α2 was obtained at 37°C when KIF15 was present or down-regulated. Presumably, trapping of integrin at low temperatures is required as it was shown for integrin β1. On the other hand, the role of KIF15 in Dab2 distribution was further demonstrated by 2.7-fold more frequent accumulation of intracellular Dab2 in the perinuclear region following the depletion of KIF15 (Fig. 7D), pointing out that KIF15 might play a role in the re-cycling. Furthermore, elevated levels of KIF15 could increase surface level of Dab2 and, consequently, promote integrin α2 internalization. Indeed, over-expression of GFP-tagged KIF15 has increased efficiency of integrin α2 internalization nearly by 3-fold after 5 min of incubation, whereas transferrin internalization remained not changed. Altogether, these data show that KIF15 is required for cell surface localization of Dab2 which, in turn, might regulate internalization of integrin α2.

### Discussion:

Defective endocytosis of integrins leads to numerous alterations in signaling, cell cycle progression, interaction to the ECM and cytoskeleton, and, consequently, underlies crucial processes of cancer initiation, progression and metastasis. Integrin α2β1 is known as a potent suppressor of cancer metastasis, however, little is known about endocytic trafficking of this heterodimer and its potential routing into the lysosome. Herein, a systematic search for potential regulators of integrin α2 endocytic trafficking was performed by fluorescent microscopy-based RNAi assay (Figs. 1F, G, 3B). In this study, endocytic trafficking of endogenous integrin α2 in HeLa cells that is internalized in a clathrin and caveolin-dependent manner (Fig. 1F, G), passes Rab5-specific compartment and is potentially re-cycled to the plasma membrane via Rab4- and Rab11- specific structures (Fig. 2) was investigated. This route differs strongly from the degradation pathway taken by antibody-clustered integrin α2 (Fig. 1 C).

386 genes associated to membrane traffic and cytoskeleton organization have been tested and 122 were scored as potential regulators of integrin α2 trafficking (Fig. 3A). Predominantly, a reduction in integrin α2 intracellular accumulation was recorded (for 115 potential regulators), and only 7 molecules increased intracellular accumulation of integrin α2 when down-regulated. These effects might be a direct consequence of changes in membrane trafficking, but might also manifest changes in degradation and, as assessed in the validation procedure, in integrin α2 expression levels. 83 primary hits of the screen might be specific to intracellular accumulation of integrin α2 as they were not identified as regulators of transferrin or EGF endocytosis (Fig. 3C). Possibly, it might reflect differences between data acquisition (wide field vs. confocal microscopy), read-out (monoparametric vs. multiparametric), data normalization procedures (using as reference the negative controls vs. average of all samples), and testing different siRNA libraries. It is rather difficult to compare the depletion efficiencies of proteins as different sets were tested in both studies. On the other hand, both screens were performed in HeLa cells and the quality control of images prior quantification was similar. Not a surprise, therefore, that a fairly large overlap of hits (39 out of 122) to the regulators of transferrin and EGF endocytosis (Fig. 3C) was obtained. Furthermore, nearly half of the interaction partners of integrin β1, present in the above library, were also effectors of integrin α2 trafficking. On the other hand, little overlap could be found to the proteomics analysis of interaction partners of the over-expressed and tagged integrin α2 in HT1080 cells. Potentially, that is due to a different cellular context and/ or expression levels of integrin. Surprisingly, 24 out of 122 potential regulators of integrin α2 trafficking are known as potential regulators of focal adhesion formation (Fig. 3C), indicating a functional cross-talk in trafficking of ligand non-bound and ligand-bound integrins.

For data validation, several subsets of the primary hits were chosen. One subset contains 14 functionally heterogeneous molecules that were targeted by more than two siRNAs in the primary screen. 7 of them showed an inhibitory effect as in the primary screening (Table 1). Curiously, only two of them, NF2 (Merlin) and a small GTPase ARF1, predominantly influenced integrin α2 intracellular accumulation. ARF1 is a key component of the COPI coatomer complex and one of the most important trafficking regulators. Potentially, ARF1 inhibits integrin α2 trafficking by regulating re-cycling to the plasma membrane as it was demonstrated for integrin α5. However, other functions of ARF1 cannot be excluded. NF2/Merlin might act indirectly by providing a linkage between membrane-associated proteins and the actin cytoskeleton, and, by that, regulating membrane organization. On the other hand, the Drosophila ortholog of NF2/Merlin localizes to the endocytic compartment in S2 cells. Moreover, it was reported that NF2/Merlin binds to integrin β1 in Schwan cells. Reduction of intracellular integrin α2 levels caused by the down-regulation of five other validated regulators (ABL2, ARHGAP6, ITGB1, Myo1A, PTPN11) rather accounts for a strong inhibition of integrin α2 expression level (Fig. 10B). Herewith, these molecules present attractive targets for diseases associated to integrin α2 expression levels. Except integrin β1 (ITGB1), roles of these proteins in the trafficking of integrin α2 remain to be addressed.

Another subset of potential regulators taken for validation analysis was the microtubule-based molecular motors kinesins. Systematic analysis of kinesins has been already performed in the context of cell cycle regulation and membrane trafficking, but information about their roles specifically in integrin trafficking as well as molecular details of such regulation is still sparse. No effect on integrin α2 trafficking was obtained when down-regulating a subset of kinesins that had also no effect in the primary screen (except KIF13A, which nonetheless strongly inhibited integrin α2 expression), indicating a high fidelity of the present assays. Out of the 12 kinesins that were recorded as the primary hits and were taken for the validation studies, nine were expressed in HeLa cells. Three of them (KIF15, KIF18A and KIF23) strongly inhibited integrin α2 endocytic trafficking (Table 1). The validation rate of kinesins was lower than that of the regulators targeted with the multiple siRNAs; they might present truly trafficking regulators as none of them changed the expression level of integrin α2. Indeed, KIF15 and KIF23 have been identified as potential regulators of ts-O45-G (temperature sensitive mutant of vesicular stomatitis virus) biosynthetic trafficking.

This study focused on KIF15, one of the strongest effector of integrin α2 intracellular accumulation in a course of validation. Down-regulation of KIF15 inhibited intracellular accumulation of integrin α2 as well as β1 as it is shown by several complementing approaches (Table 1, Fig. 4C, D, Fig. 5A, D) without having any effect on cell numbers in a population. The latter observation is in agreement that albeit KIF15 participates in the organization of bipolar mitotic spindles, but can be displaced by KIF11 (Eg5). In addition, KIF15 was not identified as a potential regulator of cell cycle progression in a genome-wide RNAi screen. KIF15 is expressed in interphase and shows a remarkably different localization in various cells: centrosomes in HeLa cells, actin bundles in fibroblasts and microtubules in terminally postmitotic neurons. GFP-tagged murine KIF15 localized largely to cytoplasm in HeLa cells (Fig. 4A) at low to moderate over-expression levels (Fig. 11, B, D) with occasional localization to the plasma membrane, microtubules and punctate structures. Altered expression levels of KIF15 did not induce apparent alterations of actin or microtubule cytoskeleton and centrosomes, ruling out the possibility that integrin α2 intracellular accumulation is altered due to general cytoskeleton changes (Fig. 4, E, F).

Over-expression of GFP-tagged KIF15, resistant to human siRNAs, partially rescued inhibition of integrin α2 intracellular accumulation caused by KIF15 depletion (Fig. 4A, B). In addition, increased cell surface integrin α2 expression was measured following KIF15 down-regulation (Fig. 10C). These data somewhat contradicts the directionality of this motor. For instance, down-regulation of microtubule plus end-directed motors Kif1C and KIF16B induced reduction of cell surface localization of α5β1 integrin and transferrin re-cycling, respectively. Similarly, down-regulation of KIF15 induced accumulation of transferrin at the perinuclear region (Fig. 5B), suggesting that it might function in the trafficking from the perinuclear re-cycling compartment. On contrary, internalization of EGF and transferrin was not influenced by the depletion of KIF15 (Fig. 5B, C, E, F).

Altogether, the above data indicates the possibility that KIF15 might be rather involved in the surface delivery of an entry factor specific for integrin α2, but not other cargo. Indeed, clathrin-associated sorting proteins (CLASPs) are known to regulate integrin β1 internalization, but not that of transferrin. In agreement to that, it was shown that endocytic trafficking of non-clustered integrin α2 might require not only the clathrin-independent, but also the clathrin-dependent pathway in HeLa cells (Fig. 1F, G). It is known that down-regulation of FXNPXY-signal sorting CLASPs Dab2, ARH, and Numb are required for integrin β1 internalization in diverse migrating cells as well as for disassembly of focal adhesions. The present RNAi data using a microscopy-based integrin α2 endocytosis assay largely confirms these observations with Dab2 being the most strong effector (Fig.6). Similar to endocytosis of LDLR, down-regulation of ARH also inhibited integrin α2 intracellular accumulation by 40% (Fig. 6B). In difference, no effect of ARH depletion on surface expression of integrin β1 was reported, which possibly might reflect usage of different methodologies. Depletion of Numb, the adaptor with the prevalence to integrin α5 resulted in no significant changes of integrin α2 intracellular accumulation. As Dab2 is known to inhibit internalization of non-bound integrin β1 and turned out to be the strongest effector of non-clustered integrin α2 intracellular accumulation, attention was focused on Dab2 for further analysis. It could be shown that the down-regulation of KIF15 induced re-distribution of punctate Dab2 structures from the plasma membrane (Fig. 7A, C). Most likely, these are endocytic structures as they were shown to co-localize to clathrin and AP-2 by more than 70% in HeLa, NIH3T3 and ES-2 cells. In addition, Dab-2 specific structures were shown to co-localize to unbound integrin β1 by more than 25%. It is plausible, therefore, that their displacement from the plasma membrane inhibits internalization of non-clustered integrin α2. Dab2 is re-distributed from the peripheral patches and accumulates at the perinuclear compartment following down-regulation of KIF15 (Fig. 7D), but does not co-localize to transferrin under these conditions. In agreement, ce-Dab-1, *C. elegans* ortholog of Dab2, does not co-localize to Rab11-positive structures. On the other hand, the transferrin distribution might be affected by the mislocalized Dab2, which was shown to regulate re-cycling of numerous proteins, including transferrin. Whether KIF15 influences Dab2 distribution through direct binding or indirectly remains to be answered, particularly, in a frame of cell cycle progression as Dab2 cell surface localization and function in endocytosis is changing throughout the cell cycle. Furthermore, emerging data on KIF15 over-expression in various tumors necessitates further analysis whether KIF15 and potential functional interaction with Dab2 is required for integrin trafficking in diseased or also in normal cells. Finally, determining of cargo specificity of KIF15 requires thorough studies.

Molecular mechanisms acting in endocytic trafficking of integrin α2β1, one of the most important receptor of collagens, emerges as an exciting research area. This knowledge is essential for prevention and treatment of diseases associated to integrin-mediated adhesion. Here, a microscopy-based assay was presented that is suitable for high-throughput applications and is helpful in systematic identification of novel regulators of integrin α2 intracellular accumulation and/ or expression.

### Summary:

α2β1 is one of the most important collagen binding integrins and is involved in numerous widely spread thrombotic and immune diseases. Furthermore, it is a potent tumor suppressor and its down-regulation increases metastasis and poor disease prognosis. Little is known how cell surface expression and trafficking of α2β1 integrin is regulated. Here, the impact of 386 cytoskeleton associated or regulatory genes on α2β1 integrin endocytosis was tested in a quantitative fluorescence microscopy-based RNAi assays on cell arrays, and 122 of them were identified to potentially regulate integrin α2 intracellular accumulation. 83 identified regulators might be potentially specific for integrin trafficking and/or expression, as they did not influence internalization of EGF or transferrin. The cargo specificity was further analyzed for one of the strongest validated inhibitors of α2β1 endocytic trafficking - the microtubule-based molecular motor KIF15. These data attribute a novel role of KIF15 in regulating plasma membrane localization of the alternative clathrin adaptor Dab2 and thereby specifically influencing internalization of integrin α2. In the present study, numerous novel regulators of integrin α2 endocytosis have been identified that might play important roles in diseases associated to integrin-mediated adhesion.

## Claims

1. A method for the identification of genes, gene products, ncRNAs, and/or chemical agents which modulate the expression, localization and/or trafficking of an integrin of interest, said method comprising the steps:
(a) providing a solid support carrying cells on said solid support in defined locations,
(b1) modulating the expression of one or more gene(s) of interest in said cells, wherein per defined location on the solid support one gene of interest is modulated, and/or
(b2) modulating the activity of one or more gene product(s) of interest in said cells, wherein per defined location on the solid support, the activity of one gene product of interest is modulated, and/or
(b3) modulating the expression of one or more ncRNA(s) of interest in said cells, wherein per defined location on the solid support one ncRNA of interest is modulated, and/or
(b4) applying one or more chemical agent(s) of interest to said cells, wherein per defined location on the solid support one chemical agent of interest is applied,
wherein any number of the steps (b1) to (b4) can be performed in any order and in any combination,
(c1) determining the amount of intracellular integrin of interest by
(i) contacting said cells with a primary binding agent which specifically recognizes the integrin of interest,
(ii) subjecting the cells to conditions that promote the internalization of the integrin of interest,
(iii) stripping remaining cell surface-bound primary binding agents from the cells,
(iv) labeling said cells intracellularly with a secondary binding agent which specifically binds to the primary binding agent and carries a fluorescent label, and
(v) determining the amount of the fluorescent label in the intracellular compartment of the cells in each defined location, wherein the amount of fluorescent label correlates to the amount of intracellular integrin of interest,
and/or
(c2) determining the amount of surface integrin of interest by
(i) subjecting the cells to conditions that promote the internalization of the integrin of interest,
(ii) labeling the surface of said cells with
(α) a primary binding agent which specifically recognizes the integrin of interest and carries a fluorescent label, or
(β) a primary binding agent which specifically recognizes the integrin of interest and subsequently with a secondary binding agent which specifically binds to the primary binding agent and carries a fluorescent label,
(iii) determining the amount of the fluorescent label on the surface of the cells in each defined location, wherein the amount of fluorescent label correlates to the amount of surface integrin of interest,
and/or
(c3) determining the total amount of integrin of interest by
(i) contacting said cells with a primary binding agent which specifically recognizes the integrin of interest,
(ii) subjecting the cells to conditions that promote the internalization of the integrin of interest,
(iii) labeling said cells intracellularly and on the surface with a secondary binding agent which specifically binds to the primary binding agent and carries a fluorescent label, and
(iv) determining the total amount of the fluorescent label on the surface of the cells and in the intracellular compartment of the cells in each defined location, wherein the total amount of fluorescent label correlates to the total amount of integrin of interest,
wherein any number of the steps (c1) to (c3) can be performed in any order and in any combination,
(d) comparing the amount(s) of the fluorescent label determined in steps (c1) to (c3) and/or comparing ratios of said amounts to each other for each defined location with the respective amount(s) of the fluorescent label and/or respective ratios of said amounts determined in control cells in which no modulation of gene expression, no modulation of the activity of a gene product, no modulation of the expression of an ncRNA, and no application of a chemical agent has been performed, and
(e) identifying genes and/or gene products and/or ncRNAs and/or chemical agents which modulate the expression, localization and/or trafficking of the integrin of interest, wherein such genes and/or gene products and/or ncRNAs and/or chemical agents are indicated by a statistically significant difference between the amount(s) of the fluorescent label(s) determined in steps (e1) to (e3) for each defined location and/or ratios of said amounts to each other, and the amount(s) of the fluorescent label(s) and/or respective ratios of said amounts to each other determined in control cells in which no modulation of gene expression, no modulation of the activity of a gene product, no modulation of the expression of an ncRNA, and no application of a chemical agent has been performed in each defined location.

2. The method according to claim 1, wherein the integrin is integrin α2β1.

3. The method according to claim 1 or claim 2, wherein the solid support is selected from the group consisting of multiwell plates, microtiter plates, glass slides, coverslips, micropatterned and/or bioengineered surfaces, and cell arrays.

4. The method according to any one of claims 1 to 3, wherein the solid support is coated with one or more compound(s), selected from the group consisting of native or modified components of the extracellular matrix.

5. The method according to any one of claims 1 to 4, wherein the primary and secondary binding agents used in steps (c1) to (c3) are independently selected from the group consisting of antibodies, antibody fragments, and antibody mimetics.

6. The method according to any one of claims 1 to 5, wherein steps (c1)(i) and (c3)(i) are performed at about 4°C.

7. The method according to any one of claims 1 to 6, wherein in steps (c1)(ii), (c2)(i), and (c3)(i) the cells are subjected to about 37°C for at least 5 minutes.

8. The method according to any one of claims 1 to 7, wherein in step (d) the amount of the fluorescent label(s) in each defined location is determined by wide-field microscopy, confocal microscopy, total internal reflection fluorescence (TIRF) microscopy, high-resolution microscopy or a combination thereof.

9. The method according to any one of claims 1 to 8, further comprising the step of automatically classifying cells into discrete subpopulations according to their efficiency of endocytic trafficking of the integrin of interest after steps (c1) to (c3), wherein said classification is obtained by using the Gaussian mixture model, assuming that in each subpopulation the cells are normally distributed.

10. The method according to any one of claims 1 to 9, further comprising the step of providing at least one additional stimulus to the cells at any point after step (a) and prior to steps (c1) to (c3).

11. The method of claim 10, wherein the additional stimulus is selected from the group consisting of addition of epidermal growth factor (EGF) and addition of fibroblast growth factor (FGF) to the cells.

12. The method according to any one of claims 1 to 11, wherein said method is an automated method.
